# EUROPEAN PATENT APPLICATION

(11) **EP 4 549 006 A1**
(43) Date of publication of application: **07.05.2025**
(21) Application number: 23831283.9
(22) Date of filing: 22.06.2023
(51) Int. Cl.: B01D 61/36, B01D 61/58, B01D 63/00, B01D 63/10, C12M 1/00, C12M 1/12

(54) **MEMBRANE SEPARATION DEVICE, MEMBRANE SEPARATION SYSTEM, AND METHOD FOR OPERATING MEMBRANE SEPARATION DEVICE**

(30) Priority: 28.06.2022 JP 2022103712
(71) Applicant: NITTO DENKO CORPORATION, Ibaraki-shi Osaka 567-8680 (JP)
(72) Inventor: OGAWA Tomoya, Ibaraki-shi, Osaka 567-8680 (JP); KIMURA Naomichi, Ibaraki-shi, Osaka 567-8680 (JP); HIRAI Tomoya, Ibaraki-shi, Osaka 567-8680 (JP); NAKANO Takeshi, Ibaraki-shi, Osaka 567-8680 (JP)
(74) Representative: Hoffmann Eitle
(86) International application number: PCT/JP2023/023240
(87) International publication number: WO 2024/004839

(57) **Abstract**

The present invention provides a membrane separation device suitable for suppressing deterioration of hygienic conditions inside a membrane separation device. The membrane separation device includes a spiral membrane element. The membrane element includes: a central tube having a through hole; and a membrane leaf wound around the central tube and having a pervaporation membrane that separates a fermented liquid containing a volatile organic compound into a permeated fluid and a non-permeated fluid. A flow of the non-permeated fluid into a space adjacent to an outer circumferential surface of the membrane element is blocked during operation of the membrane separation device.

## Description

### TECHNICAL FIELD

The present invention relates to a membrane separation device, a membrane separation system, and a method for operating a membrane separation device.

### BACKGROUND ART

There have been developed methods for producing a volatile organic compound (a fermented product), such as an alcohol, by fermenting a carbon source, such as glucose, by using a microorganism. The fermentation of a carbon source is carried out in an aqueous solution, for example. In this method, the fermentation by a microorganism stops in some cases when the content of the fermented product in the aqueous solution increases. In order to continue the production of the fermented product by a microorganism, it is necessary to separate the fermented product from the aqueous solution.

As an example of the method for separating a volatile organic compound from an aqueous solution containing the organic compound, a pervaporation method using a separation membrane can be mentioned. The pervaporation method is suitable for separating a volatile organic compound from an aqueous solution containing various substances. The pervaporation method also tends to be able to better suppress the amount of energy consumption and the amount of carbon dioxide emission than a distillation method. By combining a membrane separation device that performs the pervaporation method with a fermenter that produces a fermented product, it is possible to produce a fermented product continuously. For example, Patent Literature 1 discloses a membrane separation system obtained by combining a membrane separation device with a fermenter.

### CITATION LIST

### Patent Literature

Patent Literature 1: JP 2010-161987 A

### SUMMARY OF INVENTION

### Technical Problem

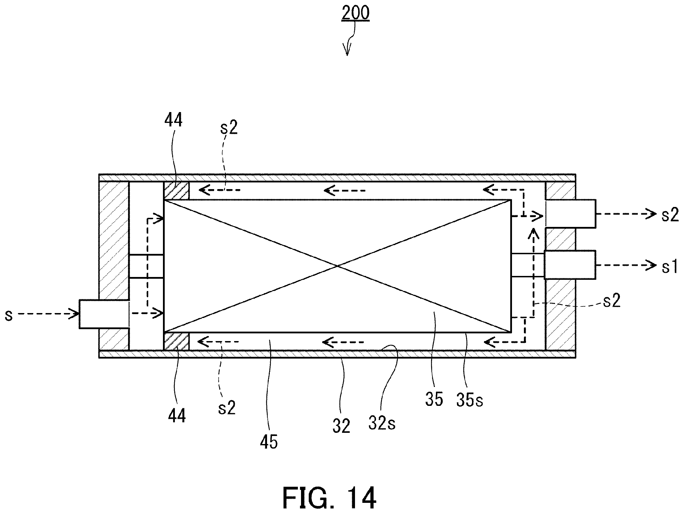

For the above-mentioned pervaporation method, a membrane separation device including a spiral membrane element that has a pervaporation membrane is used in some cases. Examples of such a membrane separation device include a membrane separation device including a spiral membrane element and a casing that accommodates the membrane element as shown in FIG. 14. In a membrane separation device 200 shown in FIG. 14, a non-permeated fluid s2 can flow into a space adjacent to an outer circumferential surface 35s of a spiral membrane element 35, specifically into a space 45 formed between the outer circumferential surface 35s and an inner circumferential surface 32s of a casing 32. When the non-permeated fluid s2 stagnates in the space 45, hygienic conditions are deteriorated, and thus it is required to suppress the stagnation as much as possible.

Therefore, the present invention is intended to provide a membrane separation device suitable for suppressing deterioration of hygienic conditions inside a membrane separation device.

### Solution to Problem

The present invention provides a membrane separation device including a spiral membrane element, wherein
the membrane element includes: a central tube having a through hole; and a membrane leaf wound around the central tube and having a pervaporation membrane that separates a fermented liquid containing a volatile organic compound into a permeated fluid and a non-permeated fluid, and
a flow of the non-permeated fluid into a space adjacent to an outer circumferential surface of the membrane element is blocked during operation.

In another aspect, the present invention provides a membrane separation device including a spiral membrane element, wherein
the membrane element includes: a central tube having a through hole; and a membrane leaf wound around the central tube and having a pervaporation membrane that separates a fermented liquid containing a volatile organic compound into a permeated fluid and a non-permeated fluid, and
a condition (1) or (2) below is satisfied:
   (1) A space adjacent to an outer circumferential surface of the membrane element is sealed;
   (2) A space adjacent to an outer circumferential surface of the membrane element forms a flow passage for a fluid other than the non-permeated fluid.

In still another aspect, the present invention provides a membrane separation system including the membrane separation device of the present invention.

In still another aspect, the present invention provides a method for operating a membrane separation device including a spiral membrane element,
the membrane element including: a central tube having a through hole; and a membrane leaf wound around the central tube and having a pervaporation membrane,
the method including:
   supplying a fermented liquid containing a volatile organic compound to the membrane separation device;
   separating the fermented liquid into a permeated fluid and a non-permeated fluid with the pervaporation membrane; and
   discharging the non-permeated fluid from the membrane separation device while blocking a flow of the non-permeated fluid into a space adjacent to an outer circumferential surface of the membrane element.

### Advantageous Effects of Invention

The present invention can provide a membrane separation device suitable for suppressing deterioration of hygienic conditions inside a membrane separation device.

### BRIEF DESCRIPTION OF DRAWINGS

FIG. 1 is a schematic cross-sectional view showing a membrane separation device of Embodiment 1.
FIG. 2 is a developed perspective view showing schematically a spiral membrane element included in the membrane separation device of the present embodiment.
FIG. 3 is a schematic cross-sectional view of the spiral membrane element of FIG. 2.
FIG. 4 is a schematic cross-sectional view of a pervaporation membrane included in the membrane separation device of the present embodiment.
FIG. 5 is a schematic cross-sectional view showing a membrane separation device of Embodiment 2.
FIG. 6 is a schematic cross-sectional view showing a membrane separation device of Embodiment 3.
FIG. 7 is a schematic cross-sectional view showing a membrane separation device of Embodiment 4.
FIG. 8 is a schematic cross-sectional view showing a membrane separation device of Embodiment 5.
FIG. 9 is a schematic cross-sectional view showing a membrane separation device of Embodiment 6.
FIG. 10 is a schematic cross-sectional view showing a membrane separation device of Embodiment 7.
FIG. 11 is a schematic cross-sectional view showing a membrane separation device of Embodiment 8.
FIG. 12 is a schematic configuration diagram showing a membrane separation system of the present embodiment.
FIG. 13A is a schematic configuration diagram showing Modification 1 of the membrane separation system of the present embodiment.
FIG. 13B is a schematic configuration diagram showing Modification 2 of the membrane separation system of the present embodiment.
FIG. 14 is a schematic cross-sectional view showing a conventional membrane separation device including a spiral membrane element.

### DESCRIPTION OF EMBODIMENTS

A membrane separation device according to a first aspect of the present invention is a membrane separation device including a spiral membrane element, wherein
the membrane element includes: a central tube having a through hole; and a membrane leaf wound around the central tube and having a pervaporation membrane that separates a fermented liquid containing a volatile organic compound into a permeated fluid and a non-permeated fluid, and
a flow of the non-permeated fluid into a space adjacent to an outer circumferential surface of the membrane element is blocked during operation.

A membrane separation device according to a second aspect of the present invention is a membrane separation device including a spiral membrane element, wherein
the membrane element includes: a central tube having a through hole; and a membrane leaf wound around the central tube and having a pervaporation membrane that separates a fermented liquid containing a volatile organic compound into a permeated fluid and a non-permeated fluid, and
a condition (1) or (2) below is satisfied:
   (1) A space adjacent to an outer circumferential surface of the membrane element is sealed;
   (2) A space adjacent to an outer circumferential surface of the membrane element forms a flow passage for a fluid other than the non-permeated fluid.

According to a third aspect of the present invention, for example, in the membrane separation device according to the first or second aspect,
the outer circumferential surface of the membrane element is composed of a flow passage material.

According to a fourth aspect of the present invention, for example, the membrane separation device according to any one of the first to third aspects further includes
a sealing part disposed around the membrane element.

According to a fifth aspect of the present invention, for example, in the membrane separation device according to the fourth aspect,
the sealing part has a through hole for allowing the fermented liquid to flow into the space.

According to a sixth aspect of the present invention, for example, in the membrane separation device according to the fourth aspect,
the space is sealed with two pieces of the sealing parts.

According to a seventh aspect of the present invention, for example, in the membrane separation device according to the sixth aspect,
the sealing part includes a casting agent.

According to an eighth aspect of the present invention, for example, in the membrane separation device according to the first or second aspect,
the space is sealed by being filled with a filler.

According to a ninth aspect of the present invention, for example, in the membrane separation device according to the first or second aspect,
the space is filled with a material with a thermal conductivity of 0.1 W/m•K or more.

According to a tenth aspect of the present invention, for example, in the membrane separation device according to any one of the first to ninth aspects,
the membrane element is configured to be detachable from the casing.

A membrane separation system according to an eleventh aspect of the present invention includes
the membrane separation device according to any one of the first to tenth aspects.

According to a twelfth aspect of the present invention, for example, the membrane separation system according to the eleventh aspect further includes
a tank that stores the fermented liquid to be supplied to the membrane separation device.

According to a thirteenth aspect of the present invention, for example, in the membrane separation system according to the eleventh or twelfth aspect,
the membrane separation system includes a plurality of the membrane separation devices, and
the plurality of the membrane separation devices are connected to each other in series or in parallel.

A method for operating a membrane separation device according to a fourteenth aspect of the present invention is a method for operating a membrane separation device including a spiral membrane element,
the membrane element including: a central tube having a through hole; and a membrane leaf wound around the central tube and having a pervaporation membrane,
the method including:
   supplying a fermented liquid containing a volatile organic compound to the membrane separation device;
   separating the fermented liquid into a permeated fluid and a non-permeated fluid with the pervaporation membrane; and
   discharging the non-permeated fluid from the membrane separation device while blocking a flow of the non-permeated fluid into a space adjacent to an outer circumferential surface of the membrane element.

A membrane separation device according to a fifteenth aspect of the present invention is a membrane separation device including a spiral membrane element and a casing that accommodates the membrane element, wherein
the membrane element includes: a central tube having a through hole; and a membrane leaf wound around the central tube and having a pervaporation membrane that separates a fermented liquid containing a volatile organic compound into a permeated fluid and a non-permeated fluid, and
the non-permeated fluid does not flow into a space between an outer circumferential surface of the membrane element and an inner circumferential surface of the casing during operation.

A membrane separation device according to a sixteenth aspect of the present invention is a membrane separation device including a spiral membrane element and a casing that accommodates the membrane element, wherein
the membrane element includes: a central tube having a through hole; and a membrane leaf wound around the central tube and having a pervaporation membrane that separates a fermented liquid containing a volatile organic compound into a permeated fluid and a non-permeated fluid, and
a condition (1) or (2) below is satisfied:
   (1) A space between an outer circumferential surface of the membrane element and an inner circumferential surface of the casing is sealed;
   (2) A space between an outer circumferential surface of the membrane element and an inner circumferential surface of the casing forms a flow passage for at least one selected from the group consisting of the fermented liquid and an outflow liquid resulted from an outflow of the fermented liquid from the outer circumferential surface of the membrane element.

A method for operating a membrane separation device according to a seventeenth aspect of the present invention is a method for operating a membrane separation device including a spiral membrane element and a casing that accommodates the membrane element,
the membrane element including: a central tube having a through hole; and a membrane leaf wound around the central tube and having a pervaporation membrane,
the method including:
   supplying a fermented liquid containing a volatile organic compound to the membrane separation device;
   separating the fermented liquid into a permeated fluid and a non-permeated fluid with the pervaporation membrane; and
   discharging the non-permeated fluid from the membrane separation device in such a manner that the non-permeated fluid does not flow into a space between an outer circumferential surface of the membrane element and an inner circumferential surface of the casing.

The present invention will be described in detail below. The following description is not intended to limit the present invention to a specific embodiment.

### <Embodiment of membrane separation device>

A membrane separation device of the present embodiment includes a spiral membrane element. The membrane element includes: a central tube having a through hole; and a membrane leaf wound around the central tube and having a pervaporation membrane that separates a fermented liquid containing a volatile organic compound into a permeated fluid and a non-permeated fluid. The membrane separation device of the present embodiment is a device that performs membrane separation, during its operation, for a fermented liquid containing a volatile organic compound by using the pervaporation membrane.

In the membrane separation device of the present embodiment, a flow of the non-permeated fluid into a space adjacent to an outer circumferential surface of the membrane element is blocked during operation. In another aspect, the membrane separation device of the present embodiment satisfies a condition (1) or (2) below:
(1) A space adjacent to an outer circumferential surface of the membrane element is sealed;
(2) A space adjacent to an outer circumferential surface of the membrane element forms a flow passage for a fluid other than the non-permeated fluid. The fluid other than the non-permeated fluid is at least one selected from the group consisting of the fermented liquid, an outflow liquid resulted from an outflow of the fermented liquid from the outer circumferential surface of the membrane element, and a heating medium for heating the membrane element.

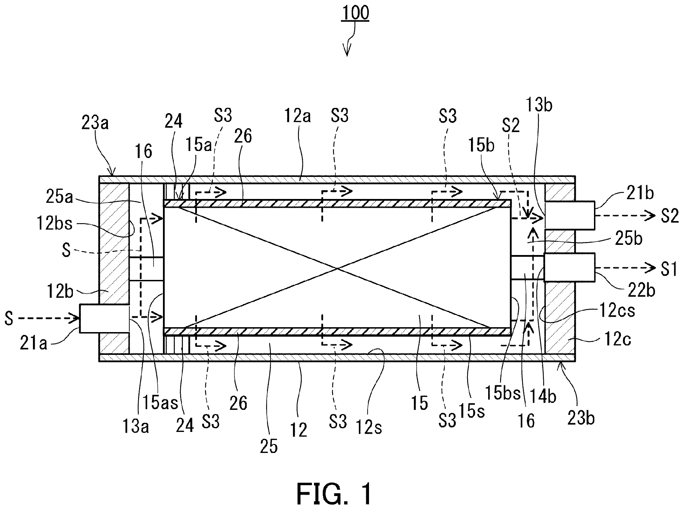

Hereinafter, Embodiments 1 to 8, which are preferable examples of the present embodiment, will be explained with reference to FIGs. 1 to 11. The elements common among Embodiments 1 to 8 are denoted by the same reference characters, and the description of such elements may be omitted. The descriptions of Embodiments 1 to 8 are applicable to each other unless technical inconsistency occurs. Furthermore, the features of Embodiments 1 to 8 may be combined with each other unless technical inconsistency occurs.

### [Embodiment 1]

FIG. 1 is a schematic cross-sectional view showing a membrane separation device 100 of Embodiment 1. As shown in FIG. 1, the membrane separation device 100 of Embodiment 1 includes a spiral membrane element 15 and a casing 12 that accommodates the membrane element 15. In the membrane separation device 100, a space 25 adjacent to an outer circumferential surface 15s of the membrane element 15 is a space formed between the outer circumferential surface 15s of the membrane element 15 and an inner circumferential surface 12s of the casing 12. In the membrane separation device 100 of Embodiment 1, the space 25 forms a flow passage for an outflow liquid S3 resulted from an outflow of the fermented liquid S from the outer circumferential surface 15s of the membrane element 15.

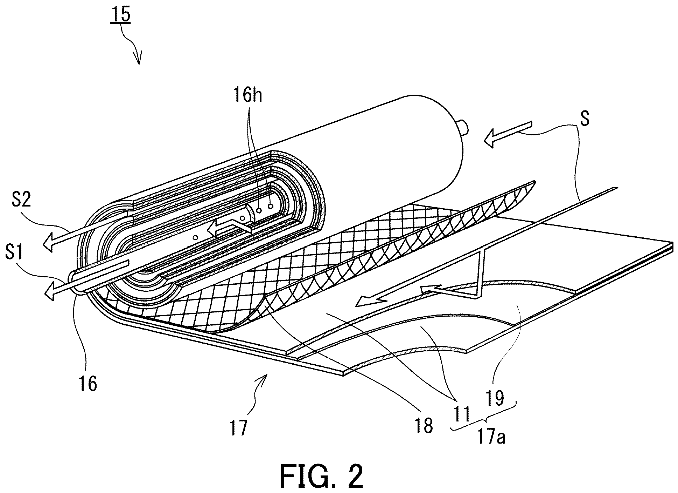

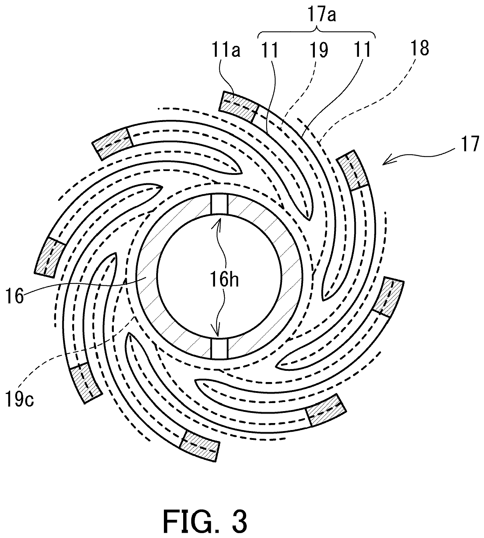

FIG. 2 is a developed perspective view showing schematically the membrane element 15 included in the membrane separation device of the present embodiment. FIG. 3 is a schematic cross-sectional view of the membrane element 15 of FIG. 2. The membrane element 15 includes: a central tube 15 having a through hole 16h; and a membrane leaf 17a wound around the central tube 15 and having a pervaporation membrane 11 that separates the fermented liquid S into a permeated fluid S1 and a non-permeated fluid S2.

As shown in FIGs. 2 and 3, the membrane element 15 includes a laminate 17. The laminate 17 is wound around the central tube 15 and disposed around the central tube 15. In an inside of the laminate 17, a feed space and a permeation space are formed.

The fermented liquid S is supplied from one end face of the laminate 17 to an inside of the membrane element 15 and flows, in parallel with a longitudinal direction of the central tube 16, through the feed space. That is, the fermented liquid S is a fluid to be supplied from an upstream-side end face of the aminate 17 included in the membrane element 15. The fluid that is a part of the fluid that flows, in parallel with the longitudinal direction of the central tube 16, through the feed space and that has flowed out from the outer circumferential surface 15s of the membrane element 15 corresponds to the outflow liquid S3. In the membrane element 15, the fermented liquid S is separated to generate the permeated fluid S1 and the non-permeated fluid S2. The permeated fluid S1 is guided to an outside via the central tube 16. The non-permeated fluid S2 is discharged to an outside of the membrane element 15 from another end face of the laminate 17. That is, the non-permeated fluid S2 is a fluid to be discharged from a downstream-side end face of the laminate 17 included in the membrane element 15.

As shown in FIG. 3, the laminate 17 has a plurality of the membrane leaves 17a. Each of the membrane leaves 17a includes the pervaporation membrane 11 and a permeation-side flow passage material 19. In FIG. 3, the permeation-side flow passage material 19 is indicated by a dashed line. Specifically, each of the membrane leaves 17a has two pieces of the pervaporation membranes 11. The two pieces of the pervaporation membranes 11 are stacked with each other and sealed on three sides in such a manner as to have a bag-like structure. For the sealing of the two pieces of the pervaporation membranes 11, an adhesive layer 11a including an adhesive is used, for example. The permeation-side flow passage material 19 is disposed between the two pieces of the pervaporation membranes 11 in such a manner as to be positioned inside the bag-like structure. The permeation-side flow passage material 19 secures, between the two pieces of the pervaporation membranes 11, a space (the permeation space) serving as a flow passage for the permeated fluid S1. As just described above, the permeation-side flow passage material 19 is combined with the pervaporation membrane 11 and used. The number of the membrane leaves 17a is, for example, but not particularly limited to, 2 to 30.

The laminate 17 further has a feed-side flow passage material 18. In FIG. 3, the feed-side flow passage material 18 is indicated by a dashed line. The feed-side flow passage material 18 is positioned outside the above-mentioned bag-like structure and is laminated on the membrane leaf 17a. Specifically, the laminate 17 has a plurality of the feed-side flow passage materials 18, and the plurality of the feed-side flow passage materials 18 and the plurality of the membrane leaves 17a are laminated alternately. The feed-side flow passage material 18 secures, between the membrane leaf 17a and the membrane leaf 17a, a space (the feed space) serving as a flow passage for the fermented liquid S.

As shown in FIGs. 2 and 3, the central tube 16 typically has a cylindrical shape, particularly a circular cylindrical shape. The central tube 16 serves the role of collecting the permeated fluid S1 having permeated through each of the pervaporation membranes 11 and guiding it to the outside of the membrane element 15. The central tube 16 is provided with the through hole 16h that allows an inner space of the central tube 16 to communicate with an outer space of the central tube 16. The through hole 16h is formed in a wall surface of the central tube 16, for example. The number of the through holes 16h is not particularly limited and may be one, or two or more. The central tube 16 may be provided with a plurality of the through holes 16h at predetermined intervals along a direction in which the central tube 16 extends. The number of rows of the through holes 16h provided along the direction in which the central tube 16 extends is not particularly limited and may be one, or two or more. The central tube 16 may be provided with two rows of the through holes 16h in such a manner that the rows face each other when viewed in cross section. An outer diameter of the central tube 16 is, for example, 10 to 100 mm, and preferably 12 to 50 mm.

Examples of a material of the central tube 16 include: a resin such as an acrylonitrile-butadiene-styrene copolymer resin (an ABS resin), a polyphenylene ether resin (a PPE resin), or a polysulfone resin (a PSF resin); and a metal such as stainless steel or titanium.

The membrane element 15 may further include a flow passage material 19c. In FIG. 3, the flow passage material 19c is indicated by a dashed line. The flow passage material 19c is positioned between the central tube 16 and the laminate 17 and is wound around the central tube 16 on a side closer to the central tube 16 than the laminate 17 is. The flow passage material 19c secures, between the laminate 17 and the central tube 16, a space serving as a flow passage for the permeated fluid S1. The flow passage material 19c is connected to an opening end of the membrane leaf 17a mentioned above. Thereby, the permeation-side flow passage material 19 of the membrane leaf 17a is connected to the flow passage material 19c. The flow passage material 19c is in contact with the through hole 16h of the central tube 16. Thereby, the permeated fluid S1 can flow from the flow passage material 19c to an inside of the central tube 16 via the through hole 16h.

As the feed-side flow passage material 18, the permeation-side flow passage material 19, and the flow passage material 19c, a resin net, woven fabric, or knitted fabric composed of polyethylene, polypropylene, polyethylene terephthalate (PET), polyphenylene sulfide (PPS), polysulfone (PSU), or an ethylene-chlorotrifluoroethylene copolymer (ECTFE) can be used, for example.

In the membrane separation device 100 of Embodiment 1, the outer circumferential surface 15s of the membrane element 15 is composed of a flow passage material 26, as shown in FIG. 1. In other words, the membrane element 15 includes the flow passage material 26 surrounding the laminate 17. Because of such a configuration, the outflow liquid S3 resulted from the outflow of the fermented liquid S from the outer circumferential surface 15s of the membrane element 15 flows into the space 25 as shown in FIG. 1. The outflow liquid S3 having flowed into the space 25 flows along the space 25. That is, the membrane separation device 100 satisfies the above-mentioned condition (2). More specifically, in the membrane separation device 100, the space 25 forms the flow passage for the outflow liquid S3. The outflow liquid S3 having flowed through the space 25 is discharged outside the membrane separation device 100 together with the fermented liquid S (the non-permeated fluid S2) not having permeated through the pervaporation membrane 11 of the membrane element 15. Therefore, a flow of the non-permeated fluid S2 into the space 25 is blocked in the membrane separation device 100.

As the flow passage material 26, those mentioned as the feed-side flow passage material 18, the permeation-side flow passage material 19, and the flow passage material 26 can be used. The flow passage material 26 may be the same as the feed-side flow passage material 18.

The membrane separation device 100 of Embodiment 1 further includes a sealing part 24 disposed around the membrane element 15. One end of the space 25 is sealed with the sealing part 24. As the sealing part 24, a sealing material with a U-shaped cross section, an O-ring sealing material, or the like can be used, for example.

In the membrane separation device 100 of Embodiment 1, the sealing part 24 is disposed at one location. In other words, one end of the space 25 is sealed with one piece of the sealing part 24. That is, in the membrane separation device 100, one piece of the sealing part 24 is used as the sealing part 24.

As shown in FIG. 1, the sealing part 24 is preferably disposed at an upstream-side end portion 15a of the membrane element 15. The sealing part 24 may be disposed slightly downstream of the upstream-side end portion 15a of the membrane element 15. Such a configuration is also expected to exert the same effects.

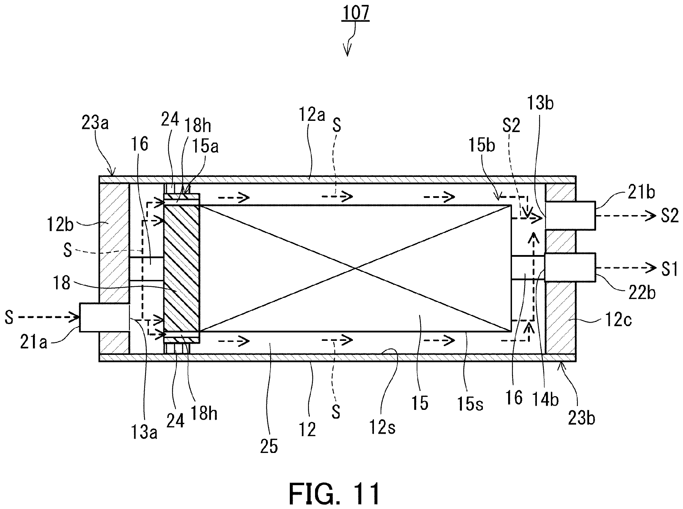

In the membrane separation device 100 of Embodiment 1, the sealing part 24 has a ring shape and surrounds the membrane element 15 in a circumferential direction. The sealing part 24 may be the same as the later-described sealing member 44 used for the conventional membrane separation device 200 shown in FIG. 11.

In the present embodiment, a shape of the casing 12 is not particularly limited as long as it can accommodate the membrane element 15. The shape of the casing 12 may be a cylindrical shape or a rectangular tube shape, for example. FIG. 1 shows an example in which the shape of the casing 12 is a cylindrical shape.

As shown in the example of FIG. 1, the casing 12 has a casing main body 12a, a first end plate 12b, and a second end plate 12c in the present embodiment. The casing main body 12a has a cylindrical shape and is made from a material having sufficient pressure resistance. The casing 12 may be a high-pressure vessel that is used for a reverse osmosis membrane. The first end plate 12b is attached to an upstream-side end portion 23a of the casing 12. The end portion 23a of the casing 12 is closed with the first end plate 12b. The second end plate 12c is attached to a downstream-side end portion 23b of the casing 12. The end portion 23b of the casing 12 is closed with the second end plate 12c.

The casing 12 has a port 21a, a port 21b, and a port 22b. These ports are ports for allowing an inside of the casing 12 to communicate with an outside of the casing 12. The port 21a is provided at the upstream-side end portion 23a of the casing 12. The port 21b and the port 22b are provided at the downstream-side end portion 23b of the casing 12.

As shown in FIG. 1, the port 21a may be provided to the first end plate 12b, and the port 22b may be provided to the second end plate 12c. The port 21b and the port 22b may be provided to the second end plate 12c.

The port 22b may be provided to the first end plate 12b. That is, the port 21a and the port 22b may be provided to the first end plate 12b.

As shown in the example of FIG. 1, an upstream-side space 25a is formed between an inner face 12bs of the first end plate 12b and an upstream-side end face 15as of the membrane element 15 in the present embodiment. A downstream-side space 25b is formed between an inner face 12cs of the second end plate 12c and a downstream-side end face 15bs of the membrane element 15. The space 25 is separated from the upstream-side space 25a by the sealing part 24.

In the membrane separation device 100, an end portion member, which is not shown, may be attached to one end or each of both ends, in an axial direction, of the membrane element 15. The end portion member is also referred to as a telescope prevention member, an anti-telescope material, and the like. In this case, the sealing part 24 seals one end of the space 25 via the end portion member disposed at the upstream-side end portion 15a of the membrane element 15. Note that in the case where the end portion member is attached to one end or each of both ends, in the axial direction, of the membrane element 15, the outer circumferential surface 15s of the membrane element 15 means to also include an outer circumferential surface of the end portion member.

In the present embodiment, the membrane element 15 is placed laterally so that a longitudinal direction thereof is along a horizontal direction. In the case where the membrane element 15 is placed laterally, the port 21b may be positioned at an upper side of the second end plate 12c. In this case, the port 21a may be positioned at a lower side of the first end plate 12b. The membrane element 15 may be placed vertically so that the longitudinal direction thereof is along a vertical direction, or may be placed obliquely so that the longitudinal direction thereof has an angle of more than 0° and less than 90° with respect to the horizontal direction.

A fermented liquid inlet 13a is an opening for supplying the fermented liquid S to the membrane separation device 100. A permeated fluid outlet 14b is an opening for discharging the permeated fluid S1 from the membrane separation device 100. A non-permeated fluid outlet 13b is an opening for discharging, from the membrane separation device 100, the fermented liquid S (the non-permeated fluid S2) not having permeated through the pervaporation membrane 11. The fermented liquid inlet 13a communicates with the port 21a, and the port 21a is used as an inlet for the fermented liquid S. The central tube 16 is connected to the port 22b at the permeated fluid outlet 14b. That is, the port 22b is used as an outlet for the permeated fluid S1. The non-permeated fluid outlet 13b communicates with the port 21b, and the port 21b is used as an outlet for the non-permeated fluid S2. Each of the ports may be a simple opening, or a nozzle-like opening as shown in FIG. 1.

In the present embodiment, the membrane element 15 may be configured to be detachable from the casing 12. In other words, the membrane element 15 may be in the form of a cartridge and configured to be replaceable for the casing 12. Such a configuration makes it possible to shorten time required to replace the membrane element 15.

In the present embodiment, the membrane element 15 may have a diameter of 8 inches (approximately 201 mm) or less. The membrane separation device including the membrane element 15 in such a size has excellent transportability.

The membrane separation device of the present embodiment may separate the fermented liquid S into the permeated fluid S1 and the non-permeated fluid S2 in a state in which the feed space of the membrane element 15 is filled with the fermented liquid S.

The membrane separation device of the present embodiment is suitable for a flow-type (continuous-type) membrane separation method. However, the membrane separation device may be used for a batch-type membrane separation method.

### (Pervaporation membrane)

The pervaporation membrane 11 is a separation membrane that allows the permeated fluid S1 that is a gas and contains the volatile organic compound to be generated by a pervaporation method. In other words, the pervaporation membrane 11 is a membrane that allows the organic compound contained in the fermented liquid S to preferentially permeate therethrough. Accordingly, a content of the organic compound in the permeated fluid S1 is higher than a content of the organic compound in the fermented liquid S. In contrast, a content of the organic compound in the non-permeated fluid S2 is lower than the content of the organic compound in the fermented liquid S.

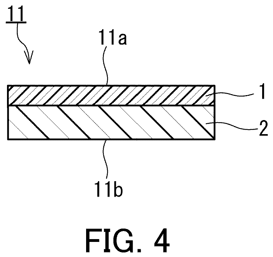

FIG. 4 is a schematic cross-sectional view of the pervaporation membrane 11 included in the membrane separation device of the present embodiment. As shown in FIG. 4, the pervaporation membrane 11 includes, for example, a separation functional layer 1 and a porous support member 2 supporting the separation functional layer 1. The pervaporation membrane 11 may further include a protective layer (not shown) that protects the separation functional layer 1. The separation functional layer 1 is in direct contact with the porous support member 2, for example. For example, the pervaporation membrane 11 has a principal surface 11a, on the separation functional layer side, that is exposed to the feed space and a principal surface 11b, on the porous support member side, that is exposed to the permeation space.

### (Separation functional layer)

The separation functional layer 1 is a layer that allows the organic compound contained in the fermented liquid S to preferentially permeate therethrough. The separation functional layer 1 includes a hydrophobic material, for example. In the present description, the term "hydrophobic material" refers to, for example, a material that has a static contact angle exceeding 90° with respect to water when a 10 µL drop of the water (temperature 25°C) is dropped on a surface of a specimen composed of the material. Note that the static contact angle with respect to water can be measured using a commercially available contact angle meter.

Examples of the hydrophobic material include a compound having a siloxane bond (a Si-O-Si bond), an olefin-based polymer, an oil, and a fluorine-based compound. The separation functional layer 1 preferably includes a compound having a siloxane bond as the hydrophobic material. The compound having a siloxane bond is typically a silicone-based polymer. The silicone-based polymer may be a solid or a liquid at 25°C. Specific examples of the silicone-based polymer include polydimethylsiloxane (PDMS). Specific examples of the olefin-based polymer include polyethylene and polypropylene. Examples of the oil include a hydrocarbon-based oil such as liquid paraffin. Examples of the fluorine-based compound include polytetrafluoroethylene (PTFE), polyvinylidene fluoride (PVDF), and tetrafluoroethylene-perfluoroalkyl vinyl ether copolymer (PFA). These hydrophobic materials can be used alone or two or more of them can be used in combination.

The separation functional layer 1 may include the hydrophobic material as a main component, or may be composed substantially of the hydrophobic material alone. The term "main component" means a component having a largest content in the separation functional layer 1 in terms of weight ratio.

The separation functional layer 1 may include a matrix including the hydrophobic material and a filler dispersed in the matrix. The filler is buried in the matrix. In the matrix, all particles of the filler may be spaced from each other or may aggregate partially.

The filler includes, for example, an inorganic material such as zeolite, silica, or bentonite. The zeolite included in the filler is preferably a high-silica zeolite having a high ratio of silica with respect to alumina. Having high resistance to hydrolysis, the high-silica zeolite is suitably used for separating the aqueous solution S1. Examples of the high-silica zeolite to be used include HSZ (registered trademark) available from Tosoh Corporation, HiSiv (registered trademark) available from UNION SHOWA K.K., USKY available from UNION SHOWA K.K., and Zeoal (registered trademark) available from Nakamura Choukou Co., Ltd.

The filler may include a metal organic framework (MOF). The metal organic framework is also referred to as a porous coordination polymer (PCP). The metal organic framework is preferably hydrophobic. The metal organic framework includes a metal ion and an organic ligand, for example. Examples of the metal ion include a Zn ion. The organic ligand includes an aromatic ring, for example. Examples of the aromatic ring included in the organic ligand include an imidazole ring. Examples of the organic ligand include 2-methylimidazole. Specific examples of the metal organic framework include ZIF-8.

The filler is in the form of particles, for example. The term "form of particles" herein includes spherical, elliptical, flaky, and fibrous forms. An average particle diameter of the filler is, for example, but not particularly limited to, 50 µm or less, preferably 20 µm or less, and more preferably 10 µm or less. The lower limit of the average particle diameter of the filler is 0.01 µm, for example. The average particle diameter of the filler can be determined by the following method, for example. First, a cross-section of the separation functional layer 1 is observed using a transmission electron microscope. The area of a specific particle of the filler on the resulting electron microscope image is calculated by image processing. The diameter of a circle having the same area as the calculated area is regarded as the particle diameter (the diameter of the particle) of the specific filler particle. The particle diameter is calculated for any number (at least 50) of the filler particles, and the average of the calculated values is regarded as the average particle diameter of the filler.

A content of the filler in the separation functional layer 1 is, for example, 10 wt% or more, preferably 30 wt% or more, and more preferably 40 wt% or more. The upper limit of the content of the filler in the separation functional layer 1 is, for example, but not particularly limited to, 70 wt%. A content of the matrix in the separation functional layer 1 is, for example, but not particularly limited to, 30 wt% to 90 wt%.

The separation functional layer 1 has a thickness of, for example, 200 µm or less, preferably 100 µm or less, and more preferably 80 µm or less. The separation functional layer 1 may have a thickness of 1.0 µm or more, 10 µm or more, or 30 µm or more.

The separation functional layer 1 may have a microporous structure with an average pore diameter of less than 0.01 µm, but may be a dense layer having no pore on its surface.

### (Porous support member)

Examples of the porous support member 2 include: a nonwoven fabric; porous polytetrafluoroethylene; aromatic polyamide fiber; a porous metal; a sintered metal; porous ceramic; porous polyester; porous nylon; activated carbon fiber; latex; silicone; silicone rubber; a permeable (porous) polymer including at least one selected from the group consisting of polyvinyl fluoride, polyvinylidene fluoride, polyurethane, polypropylene, polyethylene, polystyrene, polycarbonate, polysulfone, polyether ether ketone, polyacrylonitrile, polyimide, and polyphenylene oxide; a metallic foam having an open cell or a closed cell; a polymer foam having an open cell or a closed cell; silica; a porous glass; and a mesh screen. The porous support member 2 may be a combination of two or more of these materials.

The porous support member 2 has an average pore diameter of 0.01 to 0.4 µm, for example. A thickness of the porous support 2 is, for example, but not particularly limited to, 10 µm or more, preferably 50 µm or more, and more preferably 100 µm or more. The thickness of the porous support 2 is, for example, 300 µm or less, and preferably 200 µm or less.

### (Protective layer)

The protective layer covers a surface of the separation functional layer 1, for example. A material of the protective layer is, for example, but not particularly limited to, a silicone resin. The material of the protective layer may be the same as or different from that of the matrix of the separation functional layer 1. A thickness of the protective layer is, for example, but not particularly limited to, 0.5 µm or more, preferably 1 µm or more, more preferably 5 µm or more, and still more preferably 10 µm or more. The thickness of the protective layer is, for example, 100 µm or less, preferably 50 µm or less, and more preferably 30 µm or less.

### (Method for producing pervaporation membrane)

The pervaporation membrane 11 can be produced by, for example, forming the separation functional layer 1 on the porous support member 2. Specifically, a coating liquid containing the materials of the separation functional layer 1 is prepared first. The coating liquid may contain, in addition to the filler, a dispersant for dispersing the filler in the coating liquid. For example, in the case where the coating liquid contains the compound having a siloxane bond, the coating liquid may further contain a catalyst for curing the compound. Next, the coating liquid is applied onto the porous support 2 to obtain a coating. The coating is dried to obtain the separation functional layer 1. Thus, the pervaporation membrane 11 is obtained.

### <Embodiment of method for operating membrane separation device>

A method for operating the membrane separation device of the present embodiment includes: supplying the fermented liquid S containing the volatile organic compound to the membrane separation device; separating the fermented liquid S into the permeated fluid S1 and the non-permeated fluid S2 with the pervaporation membrane 11; and discharging the non-permeated fluid S2 from the membrane separation device while blocking the flow of the non-permeated fluid S2 into the space 25 adjacent to the outer circumferential surface 15s of the membrane element 15.

For the membrane separation device 100 of Embodiment 1, the method for operating the membrane separation device of the present embodiment is carried out as follows, for example. First, as shown in FIG. 1, the fermented liquid S is supplied to an inside of the membrane separation device 100 via the port 21a (the fermented liquid inlet 13a). Thereafter, the fermented liquid S passes through the upstream-side space 25a to be supplied to the inside of the membrane element 15, and is brought into contact with one surface of the pervaporation membrane 11 while flowing, in parallel with the longitudinal direction of the central tube 16, through the feed space. In a state in which the fermented liquid S is in contact with the one surface of the pervaporation membrane 11, the permeation space adjacent to another surface of the pervaporation membrane 11 is decompressed. Specifically, an inside of the permeation space is decompressed via the permeated fluid outlet 14b. Decompressing the inside of the permeation space makes it possible to obtain, on the side of the other surface of the pervaporation membrane 11, the permeated fluid S1 having a high content of the organic compound. In other words, the permeated fluid S1 having permeated through the pervaporation membrane 11 is supplied to the permeation space. The permeated fluid S1 moves into the central tube 16 and is discharged outside the membrane separation device 100 via the port 22b (the permeated fluid outlet 14b). The permeated fluid S1 that is a gas is cooled in a condensation unit, etc., for example. Thereby, the permeated fluid S1 is liquefied to obtain the permeated fluid S1 that is a liquid. On the other hand, the fermented liquid S (the non-permeated fluid S2) not having permeated through the pervaporation membrane 11 is discharged outside the membrane element 15. Thereafter, the non-permeated fluid S2 is discharged outside the membrane separation device 100 via the port 21b (the non-permeated fluid outlet 13b). The non-permeated fluid S2 is typically a liquid. Here, as shown in FIG. 1, the outflow liquid S3 resulted from the outflow of the fermented liquid S from the outer circumferential surface 15s of the membrane element 15 flows into the space 25. The outflow liquid S3 having flowed into the space 25 flows along the space 25. The outflow liquid S3 flowing through the space 25 inhibits the non-permeated fluid S2 from flowing into the space 25 and stagnating inside the space 25. Together with the non-permeated fluid S2, the outflow liquid S3 having flowed through the space 25 passes through the downstream-side space 25b and is discharged outside the membrane separation device 100 via the port 21b (the non-permeated fluid outlet 13b).

FIG. 14 is a schematic cross-sectional view showing a conventional membrane separation device 200 including a spiral membrane element 35. In the conventional membrane separation device 200, an outer circumferential surface 35s of the membrane element 35 is composed of a shell (not shown) produced from a material through which an aqueous solution s cannot permeate, and one end of a space 45 between the membrane element 35 and a casing 32 is sealed with a sealing member 44 disposed in the space 45. The sealing member 44 has a ring shape and surrounds the membrane element 35 in a circumferential direction. The sealing member 44 is usually disposed at a downstream-side (left-hand side in FIG. 14) end portion of the membrane element 35.

The conventional membrane separation device 200 as shown in FIG. 14 has a problem in that a non-permeated fluid s2 discharged from a downstream-side end face of the membrane element 35 flows backwards into the space 45 and stagnates inside the space 45 during operation. The stagnation of the non-permeated fluid s2 inside the space 45 helps an undesirable microorganism to grow, and thereby hygienic conditions inside the membrane separation device 200 is deteriorated in some cases. The deterioration of hygienic conditions inside the membrane separation device 200 becomes a problem particularly when a fermented liquid containing a volatile organic compound is used as the aqueous solution s to be supplied to the membrane separation device 200.

In contrast to the above, in the method for operating the membrane separation device of the present embodiment, the non-permeated fluid S2 is discharged from the membrane separation device in such a manner as not to allow the non-permeated fluid S2 to flow into the space 25 adjacent to the outer circumferential surface 15s of the spiral membrane element 15. In the membrane separation device 100 of Embodiment 1, the outflow liquid S3 flows into the space 25 and flows along the space 25 during operation, as shown in FIG. 1. That is, the space 25 forms the flow passage for the outflow liquid S3. Thereby, the non-permeated fluid S2 is inhibited from flowing into the space 25 and stagnating inside the space 25 in the membrane separation device 100. Accordingly, the growth of the undesirable microorganism inside the membrane separation device 100 is suppressed, and thereby the deterioration of hygienic conditions inside the membrane separation device 100 is suppressed.

Moreover, in the membrane separation device 100 of Embodiment 1, a temperature of the membrane element 15 is unlikely to decrease during operation because the outflow liquid S3 flows through the space 25 and thus heat from the outside of the casing 12 is likely to be conducted to the membrane element 15. As a result, it is possible to inhibit the temperature of the membrane element 15 from decreasing and performance of the pervaporation membrane 11 from being deteriorated during operation. A temperature of the outflow liquid S3 is comparable to a temperature of the fermented liquid S to be supplied to the feed space. In one example, the temperature of the fermented liquid S to be supplied to the feed space is 15°C to 75°C.

The fermented liquid S contains, for example, the organic compound as a fermented product, and water. The organic compound contained in the fermented liquid S is not particularly limited as long as the organic compound has volatility. In the present description, the term "organic compound having volatility" refers to an organic compound that has a boiling point of 20°C to 260°C, preferably 50°C to 260°C, under an atmospheric pressure (101.325 kPa). Note that the organic compound allows, for example, an aqueous phase containing water as a main component and an organic phase having a content of the organic compound higher than that in the aqueous phase to be generated when a concentration of the organic compound is high in the aqueous solution.

The number of carbon atoms in the organic compound is, for example, but not particularly limited to, 10 or less, and may be 8 or less, 6 or less, or even 4 or less. The lower limit of the number of carbon atoms in the organic compound may be 1 or 2. The organic compound has, for example, a functional group including an oxygen atom, such as a hydroxyl group, a carbonyl group, an ether group, and an ester group. In the organic compound, the number of the functional groups including an oxygen atom is typically one.

Examples of the organic compound include an alcohol, ketone, and ester. In the case where the organic compound is an alcohol, the organic compound is likely to be compatible with water and thus unlikely to cause unevenness in an environment in its system. The alcohol may be an alkyl alcohol composed only of an alkyl group and a hydroxyl group, or may be an aryl alcohol including an aryl group and a hydroxyl group. The alkyl alcohol may be linear, branched, or cyclic. Examples of the alkyl alcohol as the organic compound include methanol, ethanol, n-propanol, isopropanol, n-butanol (BuOH), 2-butanol, isobutanol, t-butanol, and n-pentanol, and preferred is n-butanol. N-butanol is a compound that allows two phases (an aqueous phase and an organic phase) to be generated from an aqueous solution thereof when a content of the compound in the aqueous solution is around 8 wt% or more. Therefore, in the case where the organic compound is n-butanol, it is possible, by adjusting the content of the organic compound in the permeated fluid to be around 8 wt% or more, for example, to generate an aqueous phase and an organic phase in the permeated fluid after the permeated fluid is liquefied. In this case, it is possible to refine the permeated fluid easily by separating the aqueous phase and the organic phase from each other. Examples of the aryl alcohol include phenol.

The ketone may be dialkylketone composed only of an alkyl group and a carbonyl group. Examples of the dialkylketone as the organic compound include methyl ethyl ketone (MEK) and acetone.

The ester may be aliphatic alkylester composed only of an alkyl group and an ester group. Examples of the aliphatic alkylester include ethyl acetate.

Note that the organic compound is not limited to those mentioned above. The organic compound may be aromatic hydrocarbon such as benzene, toluene, or xylene.

The fermented liquid S may contain one kind of the organic compound, or two or more kinds of the organic compounds. The content of the organic compound in the fermented liquid S is, for example, 50 wt% or less, and may be 30 wt% or less, 10 wt% or less, 5 wt% or less, 2 wt% or less, or even 1 wt% or less. The lower limit of the content of the organic compound is, for example, but not particularly limited to, 0.01 wt%.

In the present embodiment, the organic compound is a fermented product generated by fermentation of a carbon source by a microorganism. That is, in the present embodiment, the fermented liquid S is a fermented liquid containing the organic compound as the fermented product.

The fermented liquid S may further contain, other than water and the organic compound, an additional component, such as a microorganism associated with the generation of the fermented product, a carbon source, a nitrogen source, or an inorganic ion. The microorganism associated with the generation of the fermented product is typically a bacterium. Examples of the carbon source include polysaccharides such as starch, and monosaccharides such as glucose.

The amount of the fermented liquid S to be supplied to the membrane separation device of the present embodiment is not particularly limited and is determined according to the processing capacity of the membrane separation device. The fermented liquid S to be supplied to the feed space may be heated in advance. As mentioned above, the fermented liquid S to be supplied to the feed space has a temperature of 15°C to 75°C.

The inside of the permeation space can be decompressed by, for example, a decompression device such as a vacuum pump. A pressure in the permeation space is, for example, 50 kPa or less, and may be 20 kPa or less, 10 kPa or less, 5 kPa or less, 3 kPa or less, or even 2 kPa or less. In the present description, the term "pressure" means an absolute pressure unless otherwise noted.

As described above, the pervaporation membrane included in the membrane separation device of the present embodiment allows the organic compound contained in the fermented liquid S to preferentially permeate therethrough. Accordingly, the permeated fluid S1 obtained by the operation of the membrane separation device has a content of the organic compound higher than that in the fermented liquid S to be supplied to the membrane separation device.

A washing operation for the membrane separation device may be executed after the operation of membrane separation is executed for a certain period of time according to the above method for operating the membrane separation device. The washing operation for the membrane separation device may be executed regularly.

The method for operating the membrane separation device of the present embodiment makes it possible, for example, to produce the permeated fluid S1 having a high content of the organic compound. In other words, the method for operating the membrane separation device of the present embodiment makes it possible to produce the organic compound as the permeated fluid S1.

### <Embodiment of method for producing organic compound>

A method for producing the organic compound of the present embodiment includes: supplying the fermented liquid S to the membrane separation device; separating the fermented liquid S into the permeated fluid S1 and the non-permeated fluid S2 with the pervaporation membrane 11 of the spiral membrane element 15; discharging the non-permeated fluid S2 from the membrane separation device while blocking the flow of the non-permeated fluid S2 into the space 25 adjacent to the outer circumferential surface 15s of the membrane element 15; and recovering the permeated fluid S1. In the method for producing the organic compound of the present embodiment, the content of the organic compound in the permeated fluid S1 is higher than the content of the organic compound in the fermented liquid S.

In the method for producing the organic compound of the present embodiment, the organic compound may be an alcohol. The method for producing the organic compound of the present embodiment makes it possible to separate efficiently a volatile alcohol from the fermented liquid S containing the alcohol.

### [Embodiment 2]

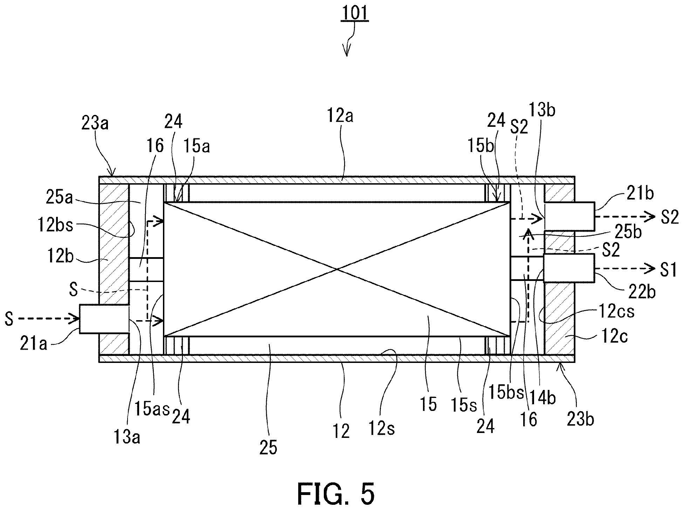

FIG. 5 is a schematic cross-sectional view showing a membrane separation device 101 of Embodiment 2. In the membrane separation device 101 of Embodiment 2, the space 25 between the outer circumferential surface 15s of the membrane element 15 and the inner circumferential surface 12s of the casing 12 is sealed. That is, the membrane separation device 101 satisfies the above-mentioned condition (1). Therefore, the flow of the non-permeated fluid S2 into the space 25 is blocked in the membrane separation device 101. Thus, the non-permeated fluid S2 is inhibited from flowing into the space 25 and stagnating inside the space 25. Accordingly, the growth of the undesirable microorganism inside the membrane separation device 101 is suppressed, and thereby the deterioration of hygienic conditions inside the membrane separation device 101 is suppressed.

In the present description, the term "the space 25 is sealed" means that the space 25 is packed, or an inlet and an outlet of the space 25 are closed.

In the membrane separation device 101 of Embodiment 2, the outer circumferential surface 15s of the membrane element 15 is composed of a shell (not shown) produced from a material through which the fermented liquid S cannot permeate. The shell may be made from FRP (fiber reinforced plastic). Therefore, unlike in the membrane separation device 100 of Embodiment 1 mentioned above, the outflow liquid S3 does not flow out from the outer circumferential surface 15s of the membrane element 15 during operation in the membrane separation device 101.

The membrane separation device 101 of Embodiment 2 includes the sealing part 24 disposed around the membrane element 15. The space 25 is sealed with the sealing part 24.

In the membrane separation device 101 of the Embodiment 2, the sealing part 24 is disposed at each of two locations. That is, two pieces of the sealing parts 24 are used as the sealing part 24 in the membrane separation device 101. One end and another end of the space 25, in other words, the inlet and the outlet of the space 25, are sealed with the two pieces of the sealing parts 24, respectively. That is, the space 25 is separated from the upstream-side space 25a and the downstream-side space 25b by the two pieces of the sealing parts 24.

As shown in FIG. 5, the two pieces of the sealing parts 24 are preferably disposed at the upstream-side end portion 15a and the downstream-side end portion 15b of the membrane element 15, respectively. Such a configuration is more likely to inhibit the non-permeated fluid S2 from flowing into the space 25 and stagnating inside the space 25. The two pieces of the sealing parts 24 may respectively be disposed slightly downstream of the upstream-side end portion 15a of the membrane element 15 and slightly upstream of the downstream-side end portion 15b of the membrane element 15. Such a configuration is also expected to exert the same effects.

The sealing part 24 has a ring shape and surrounds the membrane element 15 in the circumferential direction. The sealing part 24 may be the same as the sealing member 44 used for the conventional membrane separation device 200 shown in FIG. 11.

In the membrane separation device 101, an end portion member, which is not shown, may be attached to one end or each of both ends, in an axial direction, of the membrane element 15. In this case, the two pieces of the sealing parts 24 seal the ends of the space 25 respectively via the end portion member disposed at the upstream-side end portion 15a of the membrane element 15 and the end portion member disposed at the downstream-side end portion 15b of the membrane element 15.

In the membrane separation device 101, the space 25 may be filled with a material, which is not shown, that has high thermal conductivity. According to such a configuration, the temperature of the membrane element 15 is unlikely to decrease because heat from the outside of the casing 12 is likely to be conducted to the membrane element 15. As a result, it is possible to inhibit the temperature of the membrane element 15 from decreasing and performance of the pervaporation membrane 11 from being deteriorated during operation. Examples of the material having high thermal conductivity include water, ethylene glycol, ethanol, and a thermal paste such as a CPU grease. The thermal paste may include, for example, a matrix and thermally conductive particles dispersed in the matrix. The matrix may include silicone, for example. The conductive particles may include, for example, a metal such as aluminum. The material having high thermal conductivity may be water. The material having high thermal conductivity preferably has a thermal conductivity of 0.1 W/m•K or more. The upper limit of the thermal conductivity of the material having high thermal conductivity is not particularly limited. The upper limit is 20.0 W/m•K, for example.

For the membrane separation device 101 of Embodiment 2, the method for operating the membrane separation device of the present embodiment is carried out as follows, for example. First, as shown in FIG. 5, the fermented liquid S is supplied to an inside of the membrane separation device 101 via the port 21a (the fermented liquid inlet 13a). Thereafter, the fermented liquid S passes through the upstream-side space 25a to be supplied to the inside of the membrane element 15, and is brought into contact with one surface of the pervaporation membrane 11. In a state in which the fermented liquid S is in contact with the one surface of the pervaporation membrane 11, the permeation space adjacent to another surface of the pervaporation membrane 11 is decompressed. Thereby, the permeated fluid S1 is obtained. The permeated fluid S1 moves into the central tube 16 and is discharged outside the membrane separation device 101 via the port 22b (the permeated fluid outlet 14b). Here, in the membrane separation device 101, the space 25 is sealed with the sealing part 24, and thus the non-permeated fluid S2 is inhibited from flowing into the space 25 and stagnating inside the space 25, as shown in FIG. 5. On the other hand, the fermented liquid S (the non-permeated fluid S2) having not permeated through the pervaporation membrane 11 is discharged outside the membrane element 15. Thereafter, the non-permeated fluid S2 passes through the downstream-side space 25b and is discharged outside the membrane separation device 101 via the port 21b (the non-permeated fluid outlet 13b).

### [Embodiment 3]

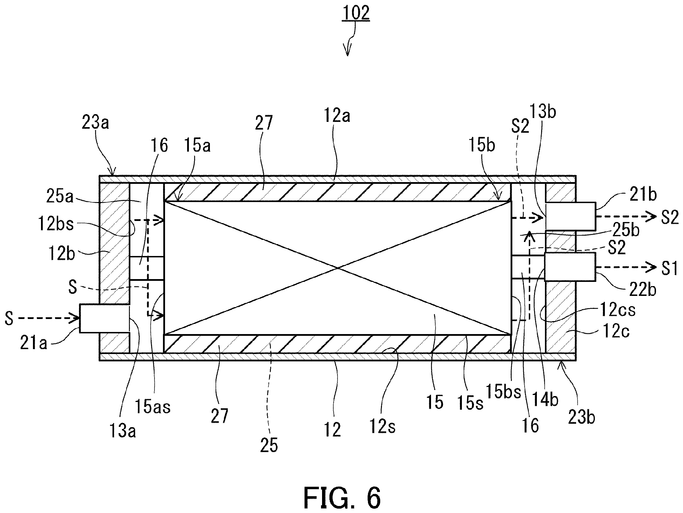

FIG. 6 is a schematic cross-sectional view showing a membrane separation device 102 of Embodiment 3. In the membrane separation device 102 of Embodiment 3, the space 25 is sealed by being filled with a filler 27. That is, the membrane separation device 102 satisfies the above-mentioned condition (1). In other words, the space 25 is packed with the filler 27. That is, the space 25 is separated from the upstream-side space 25a and the downstream-side space 25b by the filler 27. Therefore, the flow of the non-permeated fluid S2 into the space 25 is blocked in the membrane separation device 102.

As shown in FIG. 6, the filler 27 preferably fills a space between the upstream-side end portion 15a and the downstream-side end portion 15b of the membrane element 15. Such a configuration is more likely to inhibit the non-permeated fluid S2 from flowing into the space 25 and stagnating inside the space 25. The filler 27 may fill a space between slightly downstream of the upstream-side end portion 15a of the membrane element 15 and slightly upstream of the downstream-side end portion 15b of the membrane element 15. Such a configuration is also expected to exert the same effects.

The filler 27 has a ring shape and surrounds the membrane element 15 in the circumferential direction. The filler 27 allows the membrane element 15 to be fixed to the inner circumferential surface 12s of the casing 12. As the filler 27, those mentioned as the material having high thermal conductivity in Embodiment 2 may be used. According to such a configuration, the temperature of the membrane element 15 is unlikely to decrease because heat from the outside of the casing 12 is likely to be conducted to the membrane element 15. As a result, it is possible to inhibit the temperature of the membrane element 15 from decreasing and performance of the pervaporation membrane 11 from being deteriorated during operation. A commercially available filler may be used as the filler 27.

In the membrane separation device 102, an end portion member, which is not shown, may be attached to one end or each of both ends, in an axial direction, of the membrane element 15. In this case, the space 25 adjacent to the outer circumferential surface 15s of the membrane element 15 including an outer circumferential surface of the end portion member may be packed with the filler 27.

A method for operating the membrane separation device 102 of Embodiment 3 is carried out in the same manner as the method for operating the membrane separation device 101 of Embodiment 2 mentioned above.

### [Embodiment 4]

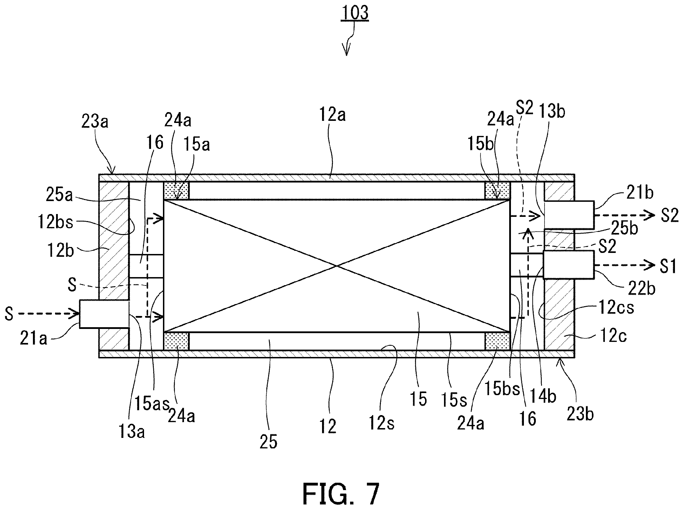

FIG. 7 is a schematic cross-sectional view showing a membrane separation device 103 of Embodiment 4. In the membrane separation device 103 of Embodiment 4, the sealing part 24 is a casting agent 24a. The membrane separation device 103 has the same configuration as that of the membrane separation device 101, except that the casting agent 24a is used as the sealing part 24. That is, the membrane separation device 103 satisfies the above-mentioned condition (1). In other words, the space 25 is separated from the upstream-side space 25a and the downstream-side space 25b by the casting agent 24a. Therefore, the flow of the non-permeated fluid S2 into the space 25 is blocked in the membrane separation device 103.

In the membrane separation device 103, the casting agent 24a allows the casing 12 to be integrated with the membrane element 15. In the present description, the word "integrated" means that components cannot be separated from each other nondestructively.

As shown in FIG. 7, the casting agent 24a is preferably disposed at each of the upstream-side end portion 15a and the downstream-side end portion 15b of the membrane element 15. Such a configuration is more likely to inhibit the non-permeated fluid S2 from flowing into the space 25 and stagnating inside the space 25. As the casting agent 24a, a urethane resin, an epoxy resin, or the like can be used, for example.

In the membrane separation device 103, an end portion member, which is not shown, may be attached to one end or each of both ends, in an axial direction, of the membrane element 15. In this case, the upstream-side casting agent 24a and the downstream-side casting agent 24a seal the ends of the space 25 respectively via the end portion member disposed at the upstream-side end portion 15a of the membrane element 15 and the end portion member disposed at the downstream-side end portion 15b of the membrane element 15.

A method for operating the membrane separation device 103 of Embodiment 4 is carried out in the same manner as the method for operating the membrane separation device 101 of Embodiment 2 mentioned above.

### [Embodiment 5]

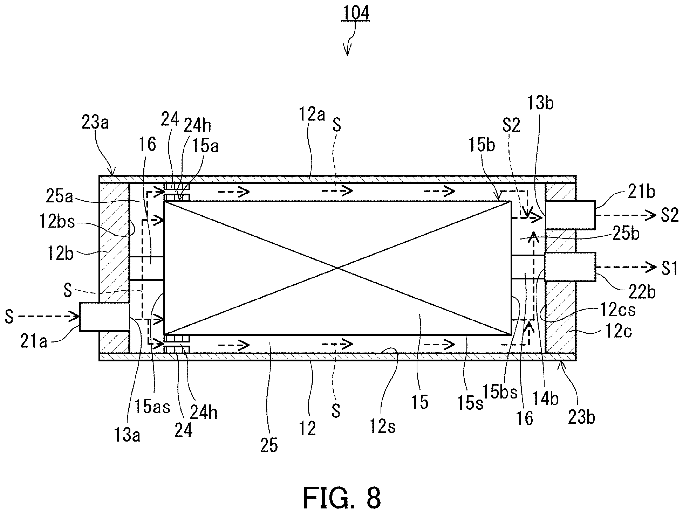

FIG. 8 is a schematic cross-sectional view showing a membrane separation device 104 of Embodiment 5. In the membrane separation device 104 of Embodiment 5, the sealing part 24 has a through hole 24h for allowing the fermented liquid S to flow into the space 35. That is, the membrane separation device 104 has a configuration in which the space 25 is not sealed with the sealing part 24.

In the membrane separation device 104 of Embodiment 5, a part of the fermented liquid S to be supplied to the membrane element 15 flows into the space 25 via the through hole 24h of the sealing part 24 as shown in FIG. 8. The fermented liquid S having flowed into the space 25 flows along the space 25. That is, the membrane separation device 104 satisfies the above-mentioned condition (2). More specifically, in the membrane separation device 104, the space 25 forms a flow passage for the fermented liquid S. The fermented liquid S having flowed through the space 25 is discharged from the non-permeated fluid outlet 13b together with the fermented liquid S (the non-permeated fluid S2) having not permeated through the pervaporation membrane 11 of the membrane element 15. Therefore, the non-permeated fluid S2 is inhibited from flowing into the space 25 and stagnating inside the space 25 in the membrane separation device 104.

Moreover, in the membrane separation device 104 of Embodiment 5, the temperature of the membrane element 15 is unlikely to decrease during operation because the fermented liquid S flows through the space 25 and thus heat from the outside of the casing 12 is likely to be conducted to the membrane element 15, as shown in FIG. 8. As a result, it is possible to inhibit the temperature of the membrane element 15 from decreasing and performance of the pervaporation membrane 11 from being deteriorated during operation. The temperature of the fermented liquid S is 15°C to 75°C as mentioned above.

The through hole 24h is provided in such a manner as to extend through the sealing part 24 in a thickness direction. The number of the through holes 24h is not particularly limited, and may be one, or two or more. The sealing part 24 may be provided with a plurality of the through holes 24h at predetermined intervals along a circumferential direction of the sealing part 24. The number of rows of the through holes 24h provided along the circumferential direction of the sealing part 24 is not particularly limited, and may be one, or two or more. In the case where the plurality of the through holes 24h are provided, the total of cross-sectional areas of the through holes 24h is preferably 50% or less of a cross-sectional area of the sealing part 24. Such a configuration makes it possible to suppress a loss of the fermented liquid S due to an excessive flow of the fermented liquid S into the space 25.

In the membrane separation device 104 of Embodiment 5, the sealing part 24 having the through hole 24h is disposed at the upstream-side end portion 15a of the membrane element 15. The position at which the sealing part 24 having the through hole 24h is disposed is not particularly limited. The sealing part 24 having the through hole 24h may be disposed at the upstream-side end portion 15a or the downstream-side end portion 15b of the membrane element 15. The sealing part 24 having the through hole 24h may be disposed at an optional position between the upstream-side end portion 15a and the stream-side end portion 15b of the membrane element 15.

The sealing part 24 having the through hole 24h may be disposed at each of two locations. As the sealing part 24, two pieces of the sealing parts 24 having the through hole 24h may be used. The two pieces of the sealing parts 24 having the through hole 24h may respectively be disposed at the upstream-side end portion 15a and the downstream-side end portion 15b of the membrane element 15.

In the membrane separation device 104, an end portion member, which is not shown, may be attached to one end or each of both ends, in an axial direction, of the membrane element 15. In this case, the sealing part 24 having the through hole 24h forms a flow passage without sealing one end of the space 25 via the end portion member disposed at the upstream-side end portion 15a of the membrane element 15.

For the membrane separation device 104 of Embodiment 5, the method for operating the membrane separation device of the present embodiment is carried out as follows, for example. First, as shown in FIG. 8, the fermented liquid S is supplied to an inside of the membrane separation device 104 via the port 21a (the fermented liquid inlet 13a). Thereafter, the fermented liquid S passes through the upstream-side space 25a to be supplied to the inside of the membrane element 15, and is brought into contact with one surface of the pervaporation membrane 11. In a state in which the fermented liquid S is in contact with the one surface of the pervaporation membrane 11, the permeation space adjacent to another surface of the pervaporation membrane 11 is decompressed. Thereby, the permeated fluid S1 is obtained. The permeated fluid S1 moves into the central tube 16 and is discharged outside the membrane separation device 104 via the port 22b (the permeated fluid outlet 14b). On the other hand, the fermented liquid S (the non-permeated fluid S2) having not permeated through the pervaporation membrane 11 is discharged outside the membrane element 15. Thereafter, the non-permeated fluid S2 is discharged outside the membrane separation device 104 via the port 21b (the non-permeated fluid outlet 13b). Here, as shown in FIG. 8, a part of the fermented liquid S to be supplied to the membrane element 15 flows into the space 25 via the through hole 24h of the sealing part 24 and flows along the space 25. That is, the space 25 forms the flow passage for the fermented liquid S. The fermented liquid S flowing through the space 25 inhibits the non-permeated fluid S2 from flowing into the space 25 and stagnating inside the space 25. Together with the non-permeated fluid S2, the fermented liquid S having flowed through the space 25 passes through the downstream-side space 25b and is discharged outside the membrane separation device 104 via the port 21b (the non-permeated fluid outlet 13b).

### [Embodiment 6]

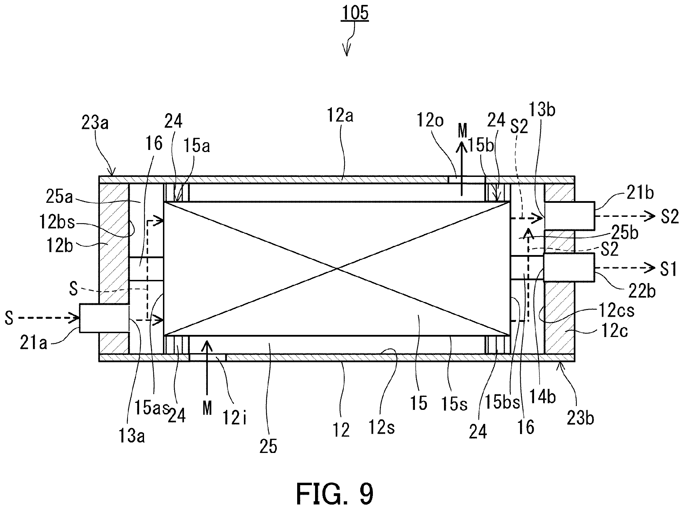

FIG. 9 is a schematic cross-sectional view showing a membrane separation device 105 of Embodiment 6. In the membrane separation device 105 of Embodiment 6, the casing 12 has an opening 12i and an opening 12o that allow the space 25 to communicate with an outside of the membrane separation device 105. The membrane separation device 105 has the same configuration as that of the membrane separation device 101, except that the casing 12 has the opening 12i and the opening 12o.

In the casing 12, the opening 12i and the opening 12o are formed respectively at positions facing the space 25.

The opening 12i functions as an inlet for a heating medium M. That is, the membrane separation device 105 satisfies the above-mentioned condition (2). More specifically, the space 25 forms a flow passage for the heating medium M in the membrane separation device 105. Therefore, the flow of the non-permeated fluid S2 into the space 25 is blocked in the membrane separation device 105. The opening 12i may be connected to a heating medium feed passage (not shown) for supplying the heating medium M to the space 25. The opening 12o functions as an outlet for the heating medium M. The opening 12o may be connected to a heating medium discharge passage (not shown) for discharging the heating medium M from the space 25. Such a configuration allows the heating medium M to flow through the space 25, so that the membrane element 15 can be heated easily. Thereby, it is possible to inhibit the temperature of the membrane element 15 from decreasing and performance of the pervaporation membrane 11 from being deteriorated during operation.

In the example shown in FIG. 9, the heating medium M is introduced to the space 25 via the opening 12i. The heating medium M introduced to the space 25 exchanges heat with the membrane element 15 via the outer circumferential surface 15s of the membrane element 15 and heats the membrane element 15. The heating medium M having exchanged heat with the membrane element 15 is discharged from the space 25 via the opening 12o. The membrane separation device 105 may be configured in such a manner that the heating medium discharge passage is connected to the heating medium feed passage and the heating medium M circulates through the heating medium feed passage, the space 25, and the heating medium discharge passage.

The heating medium M is typically warm water. However, the heating medium M may be steam (such as water vapor). The steam may be used under a pressure equal to or higher than an atmospheric pressure of an ambient environment, or may be used under a pressure lower than the atmospheric pressure of the ambient environment. In the present description, the steam used under a pressure lower than the atmospheric pressure of the ambient environment may be referred to as vacuum steam. A temperature of the heating medium M may be comparable to the temperature of the fermented liquid S to be supplied to the membrane separation device 105.

### [Embodiment 7]

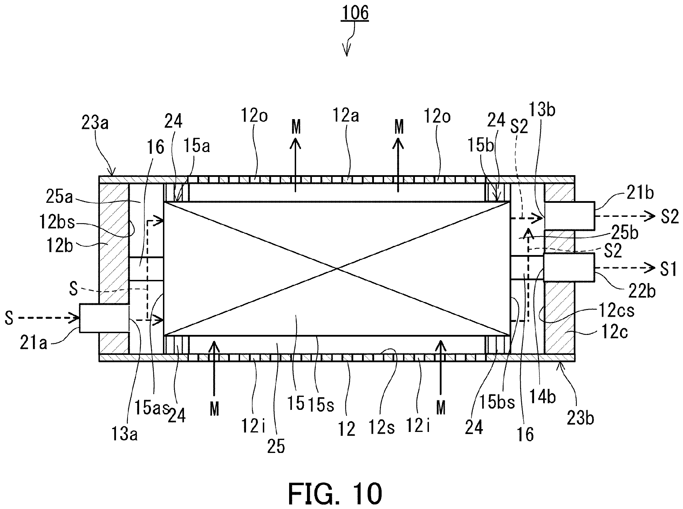

FIG. 10 is a schematic cross-sectional view showing a membrane separation device 106 of Embodiment 7. In the membrane separation device 106 of Embodiment 7, the casing 12 has a plurality of the openings 12i and a plurality of the openings 12o. The membrane separation device 106 has the same configuration as that of the membrane separation device 105, except that the casing 12 has the plurality of the openings 12i and the plurality of the openings 12o. That is, the membrane separation device 106 satisfies the above-mentioned condition (2). More specifically, the space 25 forms the flow passage for the heating medium M in the membrane separation device 106. Therefore, the flow of the non-permeated fluid S2 into the space 25 is blocked in the membrane separation device 106.

The membrane separation device 106 of Embodiment 7 makes it possible to introduce the heating medium M to the space 25 more evenly and to discharge the heating medium M from the space 25 more evenly. This makes it possible to heat the membrane element 15 evenly.

The locations of the plurality of the openings 12i and the plurality of the openings 12o are not particularly limited. In the casing, the plurality of the openings 12i and the plurality of the openings 12o may be formed evenly 12 at positions facing the space 25, as shown in FIG. 10. The number of the openings 12i and that of the openings 12o are not particularly limited. For example, the casing 12 may be configured to have the plurality of the openings 12i and the plurality of the openings 12o by having a mesh structure at each of the positions facing the space 25.

### [Embodiment 8]

FIG. 11 is a schematic cross-sectional view showing a membrane separation device 107 of Embodiment 8. The membrane separation device 107 of Embodiment 8 has at least an end portion member 18 attached to 15a of the membrane element 15, and the end portion member 18 has a through hole 18h for allowing the fermented liquid S to flow into the space 35. The membrane separation device 107 has the same configuration as that of the membrane separation device 104, except that the end portion member 18, instead of the sealing part 24, has the through hole 18h as described above. That is, the membrane separation device 107 satisfies the above-mentioned condition (2). More specifically, the space 25 forms the flow passage for the fermented liquid S in the membrane separation device 107.

In the membrane separation device 107 of Embodiment 8, a part of the fermented liquid S to be supplied to the membrane element 15 flows into the space 25 via the through hole 18h of the end portion member 18, as shown in FIG. 11. The fermented liquid S having flowed into the space 25 flows along the space 25. That is, the space 25 forms the flow passage for the fermented liquid S. The fermented liquid S is discharged from the non-permeated fluid outlet 13b together with the fermented liquid S (the non-permeated fluid S2) having not permeated through the pervaporation membrane 11 of the membrane element 15. Therefore, the non-permeated fluid S2 is inhibited from flowing into the space 25 and stagnating inside the space 25 in the membrane separation device 107.

Moreover, in the membrane separation device 107 of Embodiment 8, the temperature of the membrane element 15 is unlikely to decrease during operation because the fermented liquid S flows through the space 25 and thus heat from the outside of the casing 12 is likely to be conducted to the membrane element 15, as shown in FIG. 11. The temperature of the fermented liquid S is 15°C to 75°C as mentioned above.

### [Other embodiments]

In the membrane separation devices of Embodiments 1 to 8, the fermented liquid inlet 13a, the non-permeated fluid outlet 13b, and the permeated fluid outlet 14b are provided in a direction parallel to an axis of the central tube 16, as shown in FIG. 1 and FIG. 5 to FIG. 11. Therefore, it is easy to connect in series the membrane separation devices of Embodiments 1 to 8, in combination of the same type as or different type from each other, and use them.

### <Embodiment of membrane separation system>

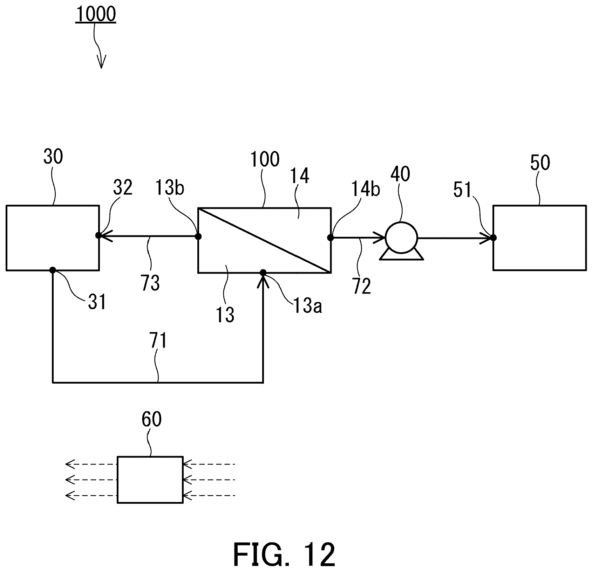

FIG. 12 is a schematic configuration diagram showing an example of a membrane separation system 1000 of the present embodiment. As shown in FIG. 12, the membrane separation system 1000 of the present embodiment includes the above-mentioned membrane separation device of the present embodiment as a membrane separation device. Hereinafter and in FIG. 12, the membrane separation device of the present embodiment is referred to as the membrane separation device 100 for convenience. The membrane separation system 1000 can carry out the above-mentioned operating method for the membrane separation device 100.

The membrane separation system 1000 further includes a tank 30 in addition to the membrane separation device 100. The tank 30 stores the fermented liquid S to be supplied to the membrane separation device 100. The tank 30 is typically a fermenter for generating the organic compound by fermentation of a carbon source by a microorganism.

The membrane separation system 1000 may further include a decompression device 40. The decompression device 40 can decompress an inside of a permeation space 14 of the membrane separation device 100. Preferably, the decompression device 40 is a vacuum device such as a vacuum pump. The vacuum pump is typically a gas transport vacuum pump, and a reciprocating vacuum pump and a rotary vacuum pump, etc. can be mentioned. Examples of the reciprocating vacuum pump include a diaphragm vacuum pump and a rocking piston vacuum pump. Examples of the rotary vacuum pump include: a liquid seal pump; an oil rotary pump (a rotary pump); a mechanical booster pump; and various kinds of dry pumps such as a roots dry pump, a claw dry pump, a screw dry pump, a turbo dry pump, and a scroll dry pump. The pump as the decompression device 40 may include a variable speed mechanism for changing a rotational speed, etc. An example of the variable speed mechanism is an inverter that drives a motor of the pump. By controlling the rotational speed, etc. of the pump by the variable speed mechanism, it is possible to adjust properly a pressure in a feed space 13 of the membrane separation device 100.

The membrane separation system 1000 may further include a recovery unit 50 for recovering the permeated fluid S1. The recovery unit 50 recovers the permeated fluid S1 delivered from the membrane separation device 100 and can, for example, store the permeated fluid S1. The recovery unit 50 is, for example, a tank that stores the permeated fluid S1.

The membrane separation system 1000 further includes a fermented liquid feed passage 71, a permeated fluid discharge passage 72, and a non-permeated fluid discharge passage 73.

The fermented liquid feed passage 71 is a passage connected to a fermented liquid outlet (an outlet 31) of the tank 30 and the fermented liquid inlet (the inlet 13a) of the membrane separation device 100 and configured to supply the fermented liquid S from the tank 30 to the membrane separation device 100. The fermented liquid feed passage 71 may be provided with a pump that controls a flow rate of the fermented liquid S, and may be provided with a sensor for measuring the content of the organic compound in the fermented liquid S.

The permeated fluid discharge passage 72 is a passage connected to the permeated fluid outlet (the outlet 14b) of the membrane separation device 100 and a permeated fluid inlet (an inlet 51) of the recovery unit 50 and configured to deliver the permeated fluid S1 from the membrane separation device 100 to the recovery unit 50. The permeated fluid discharge passage 72 may be provided with a sensor for measuring the content of the organic compound in the permeated fluid S1.

The non-permeated fluid discharge passage 73 is a passage connected to the non-permeated fluid outlet (the outlet 13b) of the membrane separation device 100 and configured to discharge the non-permeated fluid S2 from the membrane separation device 100. The non-permeated fluid discharge passage 73 may be provided with a sensor for measuring the content of the organic compound in the non-permeated fluid S2.

The non-permeated fluid discharge passage 73 may be connected to a non-permeated fluid inlet (an inlet 32) of the tank 30 and configured to deliver the non-permeated fluid S2 to the tank 30. That is, the membrane separation system 1000 may be configured to allow the non-permeated fluid S2 to be mixed with the fermented liquid S in the tank 30 and circulate through the fermented liquid feed passage 71 and the non-permeated fluid discharge passage 73. In the case where the non-permeated fluid S2 is delivered to the tank 30, the fermented liquid S is mixed with the non-permeated fluid S2 and the content of the organic compound in the fermented liquid S decreases in the tank 30. In the case where the tank 30 is a fermenter, a decrease in the content of the organic compound in the fermented liquid S can inhibit the fermentation by a microorganism from stopping, thereby making it possible to produce the fermented product continuously. In addition, since the stagnation of the non-permeated fluid S2 is suppressed in the membrane separation device 100, entry of an impurity into the tank 30 due to the stagnation can be suppressed. Moreover, accumulation of air in the space 25 of the membrane separation device 100 is also suppressed, and thus air entrainment of the membrane separation device 100 can be suppressed.

The permeated fluid discharge passage 72 may be further provided with a condensation unit for condensing the permeated fluid S1. The condensation unit is, for example, a heat exchanger for cooling the permeated fluid S1. The heat exchanger makes it possible to cool and condense the permeated fluid S1 that is a gas. The heat exchanger is, for example, a gas-liquid heat exchanger that causes heat exchange between a cooling medium, such as an antifreeze, and the permeated fluid S1 that is a gas. The condensation unit may be positioned between the membrane separation device 10 and the decompression device 40 (upstream of the decompression device 40), or between the decompression device 40 and the recovery unit 50 (downstream of the decompression device 40).

The membrane separation system 1000 may further include a controller 60 that controls each member of the membrane separation system 1000. The controller 60 is, for example, a DSP (Digital Signal Processor) including an A/D conversion circuit, an input/output circuit, an arithmetic circuit, a storage device, etc. A program for operating properly the membrane separation system 1000 is stored in the controller 60. For example, the controller 60 can control behaviors of the decompression device 40 and the like and switch between the operation of membrane separation and the washing operation.

Each passage of the membrane separation system 1000 is composed of, for example, a metal or resin pipe unless otherwise noted.

### <Modifications of membrane separation device included in membrane separation system>

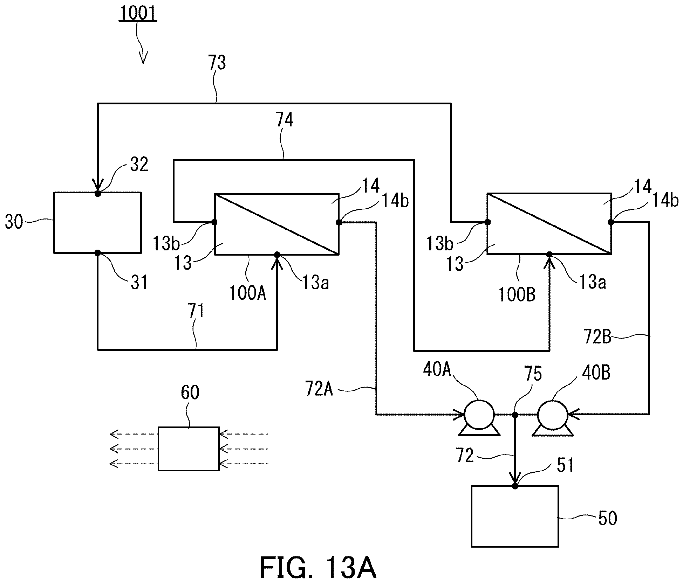

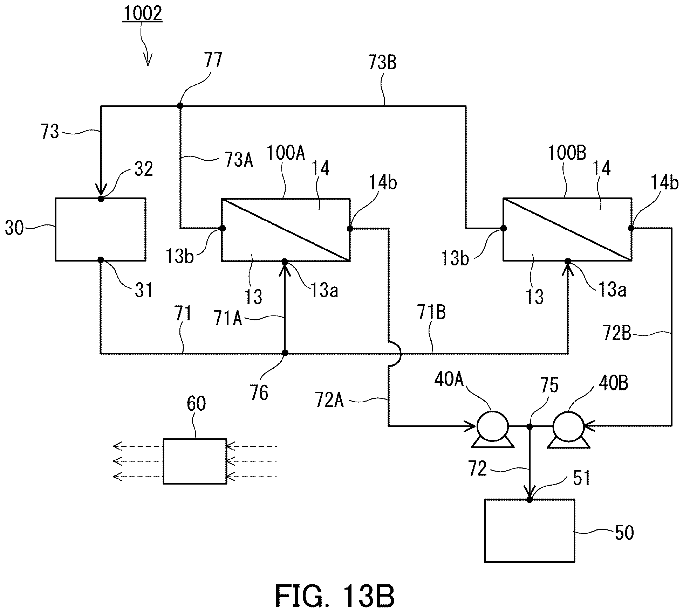

The membrane separation system 1000 of the present embodiment may include a plurality of the membrane separation devices 100, and the plurality of the membrane separation devices 100 may be connected to each other in series or in parallel. In the present description, the phrase "a plurality of the membrane separation devices 10 are connected to each other in series" refers to a configuration in which a plurality of the membrane separation devices 100 are connected to each other in such a manner that the fermented liquid S (the non-permeated fluid S2) discharged from the feed space 13 of the membrane element 15 included in the membrane separation device 100 that precedes is supplied to the feed space 13 of the membrane element 15 included in the membrane separation device 100 that follows the preceding one. The phrase "a plurality of the membrane separation devices 100 are connected to each other in parallel" refers to a configuration in which a plurality of the membrane separation devices 100 are connected to each other in such a manner that the fermented liquid S delivered from the tank 30 is supplied to the feed space 13 of the membrane element 15 included in each of the plurality of the membrane separation devices 100. The number of the membrane separation devices 100 in the membrane separation system 1000 is, for example, but not particularly limited to, 2 to 5. Hereinafter, Modifications of the membrane separation system 1000 will be explained in detail by reference to FIG. 13A and 13B.

### (Modification 1)

FIG. 13A is a schematic configuration diagram showing Modification 1 of the membrane separation system 1000 of the present embodiment. A membrane separation system 1001 of Modification 1 includes two membrane separation devices 100A and 100B connected to each other in series. The membrane separation devices 100A and 100B are connected to each other with pipes. The membrane separation system 1001 has the same configuration as that of the membrane separation system 1000, except that the membrane separation system 1001 includes the two membrane separation devices 100A and 100B, and so on. Therefore, the elements common between the above-mentioned membrane separation system 1000 and the membrane separation system 1001 of Modification 1 are denoted by the same reference numerals, and the description of such elements may be omitted. That is, the description of one embodiment is applicable to the other embodiment unless technical inconsistency occurs. Furthermore, the features of the embodiments may be combined with each other unless technical inconsistency occurs.

As described above, the membrane separation devices 100A and 100B are connected to each other in series in the membrane separation system 1001. Specifically, the membrane separation system 1001 further includes a connection passage 74 that connects the membrane separation devices 100A and 100B to each other. The connection passage 74 is connected to the non-permeated fluid outlet 13b of the membrane separation device 100A and the fermented liquid inlet 13a of the membrane separation device 100B. Note that the fermented liquid feed passage 71 is connected to the fermented liquid inlet 13a of the membrane separation device 100A, and the non-permeated fluid discharge passage 73 is connected to the non-permeated fluid outlet 13b of the membrane separation device 100B.

The permeated fluid discharge passage 72 has a first portion 72A and a second portion 72B. The first portion 72A is connected to the permeated fluid outlet 14b of the membrane separation device 100A, and the second portion 72B is connected to the permeated fluid outlet 14b of the membrane separation device 100B. The first portion 72A joins the second portion 72B at a joining point 75.

The permeated fluid discharge passage 72 is provided with two decompression devices 40A and 40B, for example. The decompression device 40A is positioned between the membrane separation device 100A and the joining point 70, and can decompress the inside of the permeation space 14 of the membrane element 15 included in the membrane separation device 100A. The decompression device 40B is positioned between the membrane separation device 100B and the joining point 70, and can decompress the inside of the permeation space 14 of the membrane element 15 included in the membrane separation device 100B. However, the permeated fluid discharge passage 72 may be provided with one decompression device, and the decompression device may be positioned between the joining point 70 and the recovery unit 50. That is, one decompression device may be shared by the membrane separation devices 100A and 100B.

Note that in the membrane separation system 1001, a pervaporation membrane 11A of the membrane element 15 included in the membrane separation device 100A may be the same as or different from a pervaporation membrane 11B of the membrane element 15 included in the membrane separation device 100B except for its membrane area. A ratio of a membrane area (m²) of the pervaporation membrane 11A with respect to a total value (m²) of the membrane area of the pervaporation membrane 11A and a membrane area of the pervaporation membrane 11B is not particularly limited.

In one example, the membrane separation system 1001 can be operated by the following method. First, a pump (not shown) is activated and the fermented liquid S is supplied from the tank 30 to the membrane separation device 100A, and the fermented liquid S is further supplied to the membrane separation device 100B from the membrane separation device 100A. Thereby, each of the pervaporation membrane 11A of the membrane element 15 included in the membrane separation device 100A and the pervaporation membrane 11B of the membrane element 15 included in the membrane separation device 100B can be brought into contact with the fermented liquid S.

Next, the permeation space 14 of the membrane element 15 included in the membrane separation device 100A is decompressed via the permeated fluid outlet 14b, and the permeation space 14 of the membrane element 15 included in the membrane separation device 100B is decompressed via the permeated fluid outlet 14b. Thereby, the operation of membrane separation can be executed by each of the membrane separation devices 100A and 100B, and the permeated fluid S1 can be obtained from each of the membrane separation devices 100A and 100B. Note that the fermented liquid S (the non-permeated fluid S2) processed by the membrane separation device 100A is delivered to the membrane separation device 100B via the connection passage 74 and further processed by the membrane separation device 100B.

Next, a pressure in the permeation space 14 of at least one of the membrane element 15 included in the membrane separation device 100A and the membrane element 15 included in the membrane separation device 100B is increased and the operation of membrane separation is ended.

### (Modification 2)

FIG. 13B is a schematic configuration diagram showing Modification 2 of the membrane separation system 1000 of the present embodiment. A membrane separation system 1002 of Modification 2 includes the two membrane separation devices 100A and 100B connected to each other in parallel. The membrane separation devices 100A and 100B are connected to each other with pipes. The membrane separation system 1002 has the same configuration as that of the membrane separation system 1000, except that the membrane separation system 1002 includes the two membrane separation devices 100A and 100B, and so on.

As described above, in the membrane separation system 1002, the membrane separation devices 100A and 100B are connected to each other in parallel. Specifically, the fermented liquid feed passage 71 has a first portion 71A and a second portion 71B. The first portion 71A of the fermented liquid feed passage 71 is connected to the fermented liquid inlet 13a of the membrane separation device 100A, and the second portion 71B is connected to the fermented liquid inlet 13a of the membrane separation device 100B. The second portion 71B is branched from the first portion 71A at a branch point 76. The branch point 76 is positioned between the tank 30 and the membrane separation device 100A.

Furthermore, the non-permeated fluid discharge passage 73 has a first portion 73A and a second portion 73B. The first portion 73A of the non-permeated fluid discharge passage 73 is connected to the non-permeated fluid outlet 13b of the membrane separation device 100A, and the second portion 73B is connected to the non-permeated fluid outlet 13b of the membrane separation device 100B. The first portion 73A joins the second portion 73B at a joining point 77. The joining point 77 is positioned between the tank 30 and the membrane separation device 100A, for example.

As in the membrane separation system 1001 of Modification 1, the permeated fluid discharge passage 72 has the first portion 72A and the second portion 72B. The first portion 72A is connected to the permeated fluid outlet 14b of the membrane separation device 100A, and the second portion 72B is connected to the permeated fluid outlet 14b of the membrane separation device 100B. The first portion 72A joins the second portion 72B at the joining point 75.

The permeated fluid discharge passage 72 is provided with the two decompression devices 40A and 40B, for example. The decompression device 40A is positioned between the membrane separation device 100A and the joining point 75, and can decompress the inside of the permeation space 14 of the membrane element 15 included in the membrane separation device 100A. The decompression device 40B is positioned between the membrane separation device 100B and the joining point 75, and can decompress the inside of the permeation space 14 of the membrane element 15 included in the membrane separation device 100B. However, the permeated fluid discharge passage 72 may be provided with one decompression device, and the decompression device may be positioned between the joining point 75 and the recovery unit 50. That is, one decompression device may be shared by the membrane separation devices 100A and 100B.

In the membrane separation system 1002, the pervaporation membrane 11A of the membrane element 15 included in the membrane separation device 100A may be the same as or different from the pervaporation membrane 11B of the membrane element 15 included in the membrane separation device 100B. The ratio of the membrane area (m²) of the pervaporation membrane 11A with respect to the total value (m²) of the membrane area of the pervaporation membrane 11A and the membrane area of the pervaporation membrane 11B is not particularly limited.

In one example, the membrane separation system 1002 can be operated by the following method. First, a pump (not shown) is activated and the fermented liquid S is supplied from the tank 30 to each of the membrane separation devices 100A and 100B. Thereby, each of the pervaporation membrane 11A of the membrane element 15 included in the membrane separation device 100A and the pervaporation membrane 11B of the membrane element 15 included in the membrane separation device 100B can be brought into contact with the fermented liquid S.

Next, the permeation space 14 of the membrane element 15 included in the membrane separation device 100A is decompressed via the permeated fluid outlet 14b, and the permeation space 14 of the membrane element 15 included in the membrane separation device 100B is decompressed via the permeated fluid outlet 14b. Thereby, the operation of membrane separation can be executed by each of the membrane separation devices100A and 100B, and the permeated fluid S1 can be obtained from each of the membrane separation devices 100A and 100B.

Next, the pressure in the permeation space 14 of at least one of the membrane element 15 included in the membrane separation device 10A and the membrane element 15 included in the membrane separation device 10B is increased and the operation of membrane separation is ended.

### INDUSTRIAL APPLICABILITY

The membrane separation system of the present embodiment is suitable for efficiently separating a volatile organic compound from a fermented liquid containing the organic compound.

## Claims

1. A membrane separation device comprising a spiral membrane element, wherein
the membrane element includes: a central tube having a through hole; and a membrane leaf wound around the central tube and having a pervaporation membrane that separates a fermented liquid containing a volatile organic compound into a permeated fluid and a non-permeated fluid, and
a flow of the non-permeated fluid into a space adjacent to an outer circumferential surface of the membrane element is blocked during operation.

2. A membrane separation device comprising a spiral membrane element, wherein
the membrane element includes: a central tube having a through hole; and a membrane leaf wound around the central tube and having a pervaporation membrane that separates a fermented liquid containing a volatile organic compound into a permeated fluid and a non-permeated fluid, and
a condition (1) or (2) below is satisfied:
(1) A space adjacent to an outer circumferential surface of the membrane element is sealed;
(2) A space adjacent to an outer circumferential surface of the membrane element forms a flow passage for a fluid other than the non-permeated fluid.

3. The membrane separation device according to claim 1 or 2, wherein the outer circumferential surface of the membrane element is composed of a flow passage material.

4. The membrane separation device according to claim 1 or 2, further comprising a sealing part disposed around the membrane element.

5. The membrane separation device according to claim 4, wherein the sealing part has a through hole for allowing the fermented liquid to flow into the space.

6. The membrane separation device according to claim 4, wherein the space is sealed with two pieces of the sealing parts.

7. The membrane separation device according to claim 6, wherein the sealing part includes a casting agent.

8. The membrane separation device according to claim 1 or 2, wherein the space is sealed by being filled with a filler.

9. The membrane separation device according to claim 1 or 2, wherein the space is filled with a material with a thermal conductivity of 0.1 W/m•K or more.

10. The membrane separation device according to claim 1 or 2, wherein the membrane element is configured to be detachable from the casing.

11. A membrane separation system comprising the membrane separation device according to claim 1 or 2.

12. The membrane separation system according to claim 11, further comprising a tank that stores the fermented liquid to be supplied to the membrane separation device.

13. The membrane separation system according to claim 11, wherein
the membrane separation system includes a plurality of the membrane separation devices, and
the plurality of the membrane separation devices are connected to each other in series or in parallel.

14. A method for operating a membrane separation device including a spiral membrane element,
the membrane element including: a central tube having a through hole; and a membrane leaf wound around the central tube and having a pervaporation membrane,
the method comprising:
supplying a fermented liquid containing a volatile organic compound to the membrane separation device;
separating the fermented liquid into a permeated fluid and a non-permeated fluid with the pervaporation membrane; and
discharging the non-permeated fluid from the membrane separation device while blocking a flow of the non-permeated fluid into a space adjacent to an outer circumferential surface of the membrane element.
